# EUROPEAN PATENT APPLICATION

(11) **EP 4 219 691 A1**
(43) Date of publication of application: **02.08.2023**
(21) Application number: 21871595.1
(22) Date of filing: 24.09.2021
(51) Int. Cl.: C12N 5/0789, A61K 35/28, A61P 35/00

(54) **USE OF COMPOUND FOR IMPROVING TRANSPLANTATION EFFICIENCY OF HUMAN HEMATOPOIETIC STEM CELLS**

(30) Priority: 24.09.2020 CN 202011015709
(71) Applicant: EdiGene (GuangZhou) Inc., Guangzhou City, Guangdong 510000 (CN)
(72) Inventor: FANG, Riguo, Beijing 102206 (CN); MA, Kuiying, Beijing 102206 (CN); LI, Chao, Beijing 102206 (CN); YUAN, Pengfei, Beijing 102206 (CN)
(74) Representative: Lavoix
(86) International application number: PCT/CN2021/120300
(87) International publication number: WO 2022/063226

(57) **Abstract**

Provided is the use of a compound for improving the transplantation efficiency of human hematopoietic stem cells, wherein the compound is selected from one of or two or more of a tubulin polymerization inhibitor, LND-212854, AZD0364, SCH772984, Pimasertib, Trametinib, IWR-1-endo, TTNPB, JNK-inhibitor IX and CHIR99021. The compound provided can improve the expression of the hematopoietic stem cell surface protein CD184 by means of in vitro short-time treatment, without affecting the phenotype, survival rate and various characteristics of the cell. By means of the binding of the chemokine receptor CD184 expressed by HSC to the chemokine ligand SDF1 in the bone marrow microenvironment, the migration ability of the HSC and the homing ability thereof in the bone marrow are improved, and the transplantation efficiency and the therapeutic effect of the HSC are enhanced.

## Description

### FIELD OF THE ART

The present application relates to the technical field of biotechnology, in particular to the use of a compound in the improvement of the transplantation efficiency of human hematopoietic stem cells.

### BACKGROUND OF THE ART

Hematopoietic stem cells (HSCs) are a type of stem cells that exist in the bone marrow and blood and have the ability to self-renew and differentiate into all types of blood cells. Based on its characteristics, hematopoietic stem cell transplantation (HSCT) is clinically considered to be the most effective, or even the only, treatment for various hematological and immune diseases.

According to the source of HSC, HSCT can be divided into autologous stem cell transplantation and allogeneic stem cell transplantation. According to the type of HSC, it can be divided into cord blood (CB), mobilized peripheral blood (mPB) and bone marrow (BM) HSC. At present, CB-HSC has received more and more attention, but the limited number of cells limits its clinical application to patients for transplantation and gene editing. Whether the number of HSCs is sufficient directly affects the success rate of transplantation, transplantation efficiency and patient recovery. In order to solve this problem, the *in vitro* culture system of HSC can be optimized from three aspects: (1) greatly increasing the number of cells expanded; (2) enhancing the efficiency of cell transplantation; (3) improving the proportion of the medium and long term hematopoietic stem cells (LT-HSC) in a unit volume, so that after the patient receives transplantation, the LT-HSC colonized in the body can rebuild the recipient's blood and immune system for a long time.

After intravenous injection, HSCs home to the bone marrow and achieve self-renewal or further differentiation through interaction with the bone marrow microenvironment. In the process of HSC migration and homing, the binding of the chemokine receptor CXCR4 (CD184) to stromal cell-derived protein (SDF-1) on the surface of HSC cells plays the most important role. One study indicates that overexpression of CD184 in human CD34+HSPCs and CD34+CD38-HSCs can significantly improve their bone marrow homing ability in an immunodeficient mouse model. Therefore, regulating the expression of CD184 has become a potential breakthrough to improve the homing of HSPCs to the bone marrow.

In recent years, several studies have found that prostaglandin E2 (PGE2), histone deacetylase inhibitors (HDACi) such asvalproic acid (VPA), glucocorticoid such as dexamethasone, hydrocortisone, fluticasone, etc. can increase its binding strength with the ligand SDF1 by regulating the expression of CD184 receptor in HSPCs , thereby playing a positive role in promotingthe migration and homing of HSPCs to bone marrow. Another study found that HSPCs treated at 39.5°C for 4 hours before transplantation can increase the expression of surfaceprotein CD184and the aggregation of surfaceprotein CD184in the lipid raft region, facilitating its interaction with SDF1, and improving the efficiency of HSCT.

In addition, some studies have found that treatmentwith mitochondrial permeation transition pore inhibitors (MPTPi), such as cyclosporin A, to avoid additional physiologic oxygen shock/stress (EPHOSS) in cellscan mimic the hypoxic environmentof HSPCs, thereby improving the transplantation efficiency of HSPCs. The rolling and residence of HSCs in the bone marrow endothelium is partly mediated by E-selectin and P-selectin. Enhanced fucosylation of E-selectin and P-selectin by treatment with guanosine diphosphate fucose and fucosyltransferase-VI also enhances HSC transplantation potential.

However, some of the currently developed methods are not completely clear about the impact on other functions of HSCs, or are limited by the difficulty of practical operation. Therefore, exploring more key molecules that can regulate the expression of CD184 on HSPCs and are expected to improve the transplantation efficiency of HSCs has become an urgent problem to be solved in hematopoietic stem cell transplantation.

### SUMMARY OF THE APPLICATION

In view of the above problems, the present application provides use of a compound for increasing CD184 protein expression on the surface of hematopoietic stem cells, the compound is one or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021, and the compounds can enhance the migration ability of HSPCs and promote the transplantation efficiency.

The specific technical solution of the present application is as follows:
1.Use of a compound in the improvement ofCD184 protein expression on the surface of hematopoietic stem cells, wherein the compound is one, three, four or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021. In some embodiments, the present application relates to use of a compound for in vitro increasing CD184 protein expression on the surface of hematopoietic stem cells, wherein the compound isone, two, three, four or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021.
2.The use according to item 1, wherein the compound is a tubulin polymerization inhibitor, preferably, the tubulin polymerization inhibitor is one, three, four or more selected from Lexibulin, Vinblastine sulfate, Colchicine and Nocodazole.
3.The use according to item 2, wherein the tubulin polymerization inhibitor is Lexibulin, Vinblastine sulfate and/or Colchicine.
4.The use according to any one of items 1-3, wherein the compound is used at a concentration of 1 nM-100 µM, such as 10 nM-50 µM, 0.1 µM-20 µM, 1 µM-10 µM, such as 0.1 µM, 0.2 µM, 0.3 µM, 0.4 µM, 0.5 µM, 0.6 µM, 0.7 µM, 0.8 µM, 0.9 µM, 1 µM, 2 µM, 3 µM, 4 µM, 5 µM, 6 µM, 7 µM, 8 µM, 9 µM, 10 µM, 20 µM, 30 µM, 40 µM, 50 µM, 60 µM, 70 µM, 80 µM, 90 µM, 100 µM, or any range therebetween.
5.Use of a compound in the improvement oftransplantation efficiency of hematopoietic stem cells, wherein the compound is one, three, four or moreselected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor. In some embodiments, the present application also relates to a method for improving the transplantation efficiency of hematopoietic stem cells, comprising supplementing a compound to a medium for culturing the hematopoietic stem cells, whereinthe compound is one, three, four or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferablythe compound is a tubulin polymerization inhibitor.
6.The use or method according to item 5, wherein the tubulin polymerization inhibitor is one, three, four or more selected from the group consisting of: Lexibulin, Vinblastine sulfate, Colchicine and Nocodazole; preferably, thetubulin polymerization inhibitor isLexibulin, Vinblastine sulfate and/or Colchicine.
7.The use according to item 5 or 6, wherein the compound is used at a concentration of 1 nM-100 µM, such as 10 nM-50 µM, 0.1 µM-20 µM, 1 µM-10 µM, such as 0.1 µM, 0.2 µM, 0.3 µM, 0.4 µM , 0.5µM, 0.6µM, 0.7µM, 0.8µM, 0.9µM, 1µM, 2µM, 3µM, 4µM, 5µM, 6µM, 7µM, 8µM, 9µM, 10µM, 20µM, 30µM, 40µM, 50µM, 60µM, 70µM, 80µM, 90 µM, 100 µM, or any range therebetween.
8.A method for increasing CD184 protein expression on the surface of hematopoietic stem cells in a subject, comprising administering to a subject in need thereof a compound, wherein the compound is one, two, there, four or more selected from the group consisting of a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
   In some embodiments, the present application also relates to a method for increasing CD184 protein expression on the surface of hematopoietic stem cells in a subject, comprising supplementing a compound to a medium for culturing the hematopoietic stem cells, wherein the compound is one, three, four or more selected from the group consisting of: atubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor agent;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
9.The method according to item 8, wherein the compound is used at a concentration of 1 nM-100 µM, such as 10 nM-50 µM, 0.1 µM-20 µM, 1 µM-10 µM, such as 0.1 µM, 0.2 µM, 0.3 µM, 0.4 µM, 0.5 µM, 0.6 µM, 0.7 µM, 0.8 µM, 0.9 µM, 1 µM, 2 µM, 3 µM, 4 µM, 5 µM, 6 µM, 7 µM, 8 µM, 9 µM, 10 µM, 20 µM, 30 µM, 40 µM, 50 µM, 60 µM, 70 µM, 80 µM, 90 µM, 100 µM or any range therebetween.
10.A method for improving transplantation efficiency of hematopoietic stem cells in a subject, comprising administering to a subject in need thereof a compound, wherein the compound is one, two, three, four or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
   In some embodiments, the present application also relates to a method for improving the transplantation efficiency of hematopoietic stem cells, comprising supplementing a compound to a medium for culturing the hematopoietic stem cells, wherein the compound is one, three, four or more selected from the group consisting of: a tubulin polymerization inhibitors, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   Preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
11.The method according to item 10, wherein the compound is used at a concentration of 1 nM-100 µM, such as 10 nM-50 µM, 0.1 µM-20 µM, 1 µM-10 µM, such as 0.1 µM, 0.2 µM, 0.3 µM, 0.4 µM, 0.5 µM, 0.6 µM, 0.7 µM, 0.8 µM, 0.9 µM, 1 µM, 2 µM, 3 µM, 4 µM, 5 µM, 6 µM, 7 µM, 8 µM, 9 µM, 10 µM, 20 µM, 30 µM, 40 µM, 50 µM, 60 µM, 70 µM, 80 µM, 90 µM, 100 µM or any range therebetween.
12.Use of a compound in the preparation of a medicament for treating a hematological malignant tumor, a hematological non-malignant tumor, a solid tumor, an immune system disease, a genetic or metabolic disease, wherein the compound is one, three, four or more selected from a group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   Preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
   A method for treating a hematological malignant tumor, a hematological non-malignant tumor, a solid tumor, an immune system disease, a genetic or metabolic disease in a subject, comprising administering to the subject hematopoietic stem cells, wherein the hematopoietic stem cells are hematopoietic stem cells cultured in the medium supplemented with a following compound which is one, three, four or more selected from the group consisting of:a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   Preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
   In some embodiments, CD184 protein expression is increased after the hematopoietic stem cells are cultured in a medium supplemented with the compound.
13.The use and method according to item 12, wherein the hematological malignant tumor is chronic myeloid leukemia, acute myeloid leukemia, acute lymphocytic leukemia, chronic myeloid leukemia, chronic lymphocytic leukemia, acute promyelocyticleukemia, non-Hodgkin's lymphoma, Hodgkin's lymphoma, myeloma, multiple myeloma, myelofibrosis and myelodysplastic syndrome, Burkitt's lymphoma, B-cell lymphoma, follicular lymphoma, mantle cell lymphoma, T-cell lymphoma, plasmablastic lymphoma, cutaneous T-cell lymphoma, primary macroglobulinemia, plasma cell leukemia, plasmablastic lymphoma, hairy cell leukemia, systemic mast cell Hyperplasia or endoplasmoid dendritic cell tumor;
   thehematological non-malignant tumor is aplastic anemia, Fanconianemia, thalassemia, sickle cell anemia, myelofibrosis, severe paroxysmal nocturnal hemoglobinuria or amegakaryocytic thrombocytopenia;
   the solid tumor is breast cancer, ovarian cancer, testicular cancer, kidney cancer, neuroblastoma, small cell lung cancer, germ cell tumor, Ewing's sarcoma, soft tissue sarcoma, Wilms tumor, osteosarcoma, medulloblastoma or malignant brain tumor;
   the immune system disease is severe combined immunodeficiency disease, severe autoimmune disease, primary central nervous system lymphoma, eczema thrombocytopenia with immunodeficiency syndrome, chronic granuloma, IPEX syndrome, AL amylosis, POEMS syndrome, hemophagocytic syndrome, rheumatoid arthritis, multiple sclerosis, systemic sclerosis, systemic lupus erythematosus, Crohn's disease, polymyositis, or dermatomyositis; and
   the genetic or metabolic diseases are mucopolysaccharidosis, dyskeratosiscongenita, lysosomal metabolic disease, spheroid leukoencephalopathy, metachromatic leukodystrophy, or X-linked adrenoleukodystrophy.
14.A medicamentfor treatinga hematological malignant tumor, malignancy hematological non-malignant tumor, a solid tumor, an immune system disease, a genetic or metabolic disease, comprising a compound,wherein the compound is one, two, three, four or more selected from the group consisting of: a tubulin polymerization inhibitor, LND- 212854, AZD0364, SCH772984, Pimasertib, Trametinib, IWR-1-endo, TTNPB, JNK-inhibitor IX and CHIR99021; preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
15.The medicament according to claim 14, wherein the hematological malignant tumor is chronic myeloid leukemia, acute myeloid leukemia, acute lymphocytic leukemia, chronic myeloid leukemia, chronic lymphocytic leukemia, acute promyelocyticleukemia, non-Hodgkin's lymphoma, Hodgkin's lymphoma, myeloma, multiple myeloma, myelofibrosis and myelodysplastic syndrome, Burkitt's lymphoma, B-Cell lymphoma, follicular lymphoma, mantle cell lymphoma T-cell lymphoma, plasmablastic lymphoma, cutaneous T-cell lymphoma, primary macroglobulinemia, plasma cell leukemia, plasmablastic lymphoma, hairy cell leukemia, systemic mastocytosis or blasticplasmacytoid dendritic cell neoplasm;
   thehematological non-malignant tumor is aplastic anemia, Fanconianemia, thalassemia, sickle cell anemia, myelofibrosis, severe paroxysmal nocturnal hemoglobinuria or amegakaryocytic thrombocytopenia;
   the solid tumor is breast cancer, ovarian cancer, testicular cancer, kidney cancer, neuroblastoma, small cell lung cancer, germ cell tumor, Ewing's sarcoma, soft tissue sarcoma, Wilms tumor, osteosarcoma, medulloblastoma or malignant brain tumor;
   the immune system disease is severe combined immunodeficiency disease, severe autoimmune disease, primary central nervous system lymphoma, eczema thrombocytopenia with immunodeficiency syndrome, chronic granuloma, IPEX syndrome, AL amylosis, POEMS syndrome, hemophagocytic syndrome, rheumatoid arthritis, multiple sclerosis, systemic sclerosis, systemic lupus erythematosus, Crohn's disease, polymyositis, or dermatomyositis; and
   the genetic or metabolic disease ismucopolysaccharidosis, dyskeratosiscongenita, lysosomal metabolic disease, spheroid leukoencephalopathy, metachromatic leukodystrophy, or X-linked adrenoleukodystrophy.
16.Use of a compound in the preparation of a medicament for treating a diseaseassociated with low expression level of surfaceprotein CD184, wherein the compound is one, three, four or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
17.Use of a compound in the preparation of a medicament for treating a disease associated with low transplantation efficiency level of hematopoietic stem cells, wherein the compound is one, three, four or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor isLexibulin, Vinblastine sulfate and/or Colchicine.
   In some of the above aspects, the present application also relates to a method for treating a disease associated with an inefficient transplantationlevel of hematopoietic stem cell, comprising administering to the subject hematopoietic stem cells, which are hematopoietic stem cells cultured in a medium supplemented with a compound, wherein the compound is one, two, three or four selected from the group consisting of: a tubulin polymerization inhibitor, LND-212854, AZD0364, SCH772984, Pimasertib, Trametinib, IWR-1-endo, TTNPB, JNK-inhibitor IX and CHIR99021 one or more, preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
   In some embodiments, the hematopoietic stem cells have increased CD184 protein expression upon culture in medium supplemented with the compound.
18. Use of a compound in the improvement ofthe homing ability of hematopoietic stem cells, wherein the compound is one, three, four or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
19.Use according to item 18, wherein the compound is used at a concentration of 1 nM-100 µM, such as 10 nM-50 µM, 0.1 µM-20 µM, 1 µM-10 µM, such as 0.1 µM, 0.2 µM, 0.3 µM, 0.4 µM, 0.5 µM, 0.6 µM, 0.7 µM, 0.8 µM, 0.9 µM, 1 µM, 2 µM, 3 µM, 4 µM, 5 µM, 6 µM, 7 µM, 8 µM, 9 µM, 10 µM, 20 µM, 30 µM, 40 µM, 50 µM, 60 µM, 70 µM, 80 µM, 90 µM, 100 µM or any range therebetween.
20.A method for improving the homing ability of hematopoietic stem cells in a subject, comprising supplementing a compound to a medium for culturing the hematopoietic stem cells, wherein the compound is one, three, four, or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021;preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
21.The method according to item 20, wherein the compound is used at a concentration of 1 nM-100 µM, such as 10 nM-50 µM, 0.1 µM-20 µM, 1 µM-10 µM, such as 0.1 µM, 0.2 µM, 0.3 µM, 0.4 µM, 0.5 µM, 0.6 µM, 0.7 µM, 0.8 µM, 0.9 µM, 1 µM, 2 µM, 3 µM, 4 µM, 5 µM, 6 µM, 7 µM, 8 µM, 9 µM, 10 µM, 20 µM, 30 µM, 40 µM, 50 µM, 60 µM, 70 µM, 80 µM, 90 µM, 100 µM or any range therebetween.
22.Use of a compound in the preparation of a medicament for treating a disease associated with low homing ability level of hematopoietic stem cells, wherein the compound is one, three, four or more selected from tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
23.A cell culture medium comprising a compound, wherein the compoundis one, three, four, or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
   preferably, the tubulin polymerization inhibitor is one, two, three, or four selected from the group consisting of: Lexibulin, Vinblastine sulfate, Colchicine and Nocodazole; preferably the tubulin polymerization inhibitor is Lexibulin, Vinblastine sulfate and/or Colchicine.
24.The cell culture mediumaccording to item 23, wherein the cell culture medium is a hematopoietic stem cell culture medium.
25.The medium according to item 23 or 24, wherein the compound is present in the medium at a concentration of 1 nM-100 µM, such as 10 nM-50 µM, 0.1 µM-20 µM, 1 µM-10 µM, such as 0.1 µM, 0.2 µM, 0.3 µM, 0.4µM, 0.5µM, 0.6µM, 0.7µM, 0.8µM, 0.9µM, 1µM, 2µM, 3µM, 4µM, 5µM, 6µM, 7µM, 8µM, 9µM, 10µM, 20µM, 30µM, 40µM, 50µM, 60µM, 70µM, 80 µM, 90 µM, 100 µM, or any range therebetween.

### The effect of the application

The compounds provided by the present application can increase protein CD184 expression on the surface of hematopoietic stem cellsthrough short-time treatment *in vitro,* without affecting the phenotype, viability and various characteristics of the cells. By combining the chemokine receptor CD184 expressed by HSC and the chemokine ligand SDF1 in the bone marrow microenvironment, the migration ability and homing ability of HSC in the bone marrow are improved, and the transplantation efficiency and therapeutic effect of HSC are enhanced.

### BRIEF DESCRIPTION OF THE DRAWINGS

Figure. 1 is a schematic diagram showingthe preliminary screening using different compounds in Example 3.
Figure. 2 is a schematic diagram showingthe secondary screening of 13 compounds in Example 3.
Figure. 3 shows the effect on CD184expressionafter treatment with Lexibulin, Vinblastine sulfate, and Tubulin polymerization inhibitor Colchicine in Example 3.
Figure. 4 is a schematic diagram showing the effect on cell viability after treatment with Lexibulin, Vinblastine sulfate, and tubulin polymerization inhibitor Colchicine in Example 4.
Figure. 5A is a schematic diagram showing the transplantation efficiency of HSPCs treated with Lexibulin, Vinblastine sulfate, and tubulin polymerization inhibitor Colchicine in the bone marrow of NPG mice in Example 5, wherein solid circles, solid squares, solid triangles and solid inverted triangles represent the detection of each mouse in each group, respectively.
Figure. 5B is a schematic diagram showing the differentiation of HSPCs treated with Lexibulin, Vinblastine sulfate, and tubulin polymerization inhibitor Colchicinein the bone marrowin Example 5.
Figure. 5C is a schematic diagram showing the differentiation of HSPCs in the spleen after treatment with Lexibulin, Vinblastine sulfate, and Tubulin polymerization inhibitor Colchicine in Example 5.
Figure.5D is a schematic diagram showing the differentiation of HSPCs treated with Lexibulin, Vinblastine sulfate, and Tubulin polymerization inhibitor Colchicinein peripheral bloodin Example 5.

### SPECIFICEMBODIMENTS

The present application will be described in detail below with reference to the embodiments described in the drawings, wherein like numerals indicate like features throughout the drawings. While specific embodiments of the present application are shown in the drawings, it should be understood that the present application may be embodied in various forms and should not be limited by the embodiments set forth herein. Rather, these embodiments are provided so that the present application will be more thoroughly understood, and will fully convey the scope of the present application to those skilled in the art.

It should be noted that certain terms are used in the description and claims to refer to specific components. It should be understood by those skilled in the art that the same component may be referred to by different nouns. The present specification and claims do not take the difference in terms as a way to distinguish components, but take the difference in function of the components as a criterion for distinguishing. As referred to throughout the specification and claims, "comprising" or "including" are open-ended terms and should be interpreted as "including but not limited to". Subsequent descriptions in the specification are preferred embodiments for implementing the presentapplication, however, the descriptions are for the purpose of general principles of the specification and are not intended to limit the scope of the present application. The scope of protection of the present application should be determined by the appended claims.

The present application provides use of a compound for increasing CD184 protein expression on the surface of hematopoietic stem cells, wherein the compound is one or more selected from tubulin polymerization inhibitor, LND-212854, AZD0364, SCH772984, Pimasertib, Trametinib, IWR-1-endo, TTNPB, JNK-inhibitor IX and CHIR99021.

CD184 is chemokine receptor CXCR4 on the surface of HSC cells. By overexpressing CD184 in human HSPCs and LT-HSCs, the bone marrow homing ability of human HSPCs and LT-HSCs can be significantly improved.

The tubulin polymerization inhibitor refers to a small molecule compound that can inhibit microtubule polymerization. Microtubules (MTs) are hollow tubular structures formed by the polymerization of two kinds of microtubulins, α-tubulin and β-tubulin, which forms protofilaments that are then highly regularly arranged. Microtubules exist in the cytoplasm and are an integral part of the cytoskeleton and participate in a variety of physiological processes, including maintenance of cell structure, intracellular material transport, cell division, cell migration, etc.

LND-212854 is an inhibitor of the BMP receptor ALK, and ALK (Activin receptor-like kinases) belongs to the transforming growth factor beta (TGFβ) family. TGFβ participates in various functions of different subpopulations of HSCs byregulating the SMAD4 transcription factor, including regulation of the key factor CD184 in HSC migration.

AZD0364 and SCH772984 are extracellular regulated protein kinase (ERK) inhibitors, which play a role in HSC growth by regulating the ERK/MEK/mTOR pathway.

Pimasertib (AS-703026) and Trametinib (GSK1120212) are MAPK (mitogen-activated protein kinase)/ERK kinase (MEK1/2) inhibitors which play a role in HSC growth by regulating the ERK/MEK/mTOR pathway.

IWR-1-endo is a Wnt pathway inhibitor. By regulating Axin2 protein, stabilizing the "destruction complex" structure formed by Axin2 and β-catenin, it promotes β-catenin phosphorylation, and thereby may enhance HSC self-renewal and differentiation potential.

TTNPB is a RAR (retinoic acid receptor) agonist, acting on human RARα, β, and γ, inhibiting their binding to [3H]tRA. Retinoids play their role through regulating transcription factors in the nucleus by the binding of ligands to RAR and retinoid X receptor (RXR). All trans-retinoic acids induce cell differentiation, and inhibition of retinoic acid receptor binding to ligands inhibits stem cell differentiation, thereby maintaining HSC sternness.

JNK-inhibitor IX is a cJun N terminal kinase (c-Jun N terminal kinase, JNK) inhibitor, involved in a variety of important physiological processes, including neural function, immune activation and embryonic development. It has been reported that JNK inhibitors can significantly increase the number of HSCs in the *in vitro* culture system, having an optimization bias for LT-HSCs, and enhancing the long term repopulating ability of HSCs.

CHIR99021 is a specific inhibitor of GSK-3α and GSK-3β, which are two isoforms of GSK-3 (Glycogen Synthase Kinase 3). GSK involves in the regulation of multiple signaling pathways such as Wnt, Hedgehog and Notch, and regulates different cell growth processes including glucose metabolism, gene transcription, and apoptosis. And GSK also plays an important role in regulating HSC homeostasis. CHIR99021 has been reported that is can promote self-renewal of human and mouse ES cells and can promote somatic cell reprogramming. It has been reported in the literature that the combination of CHIR99021 and the mTOR inhibitor Rapamycin is used to culture mouse and human HSCs, and proves that it can maintain a good long term repopulating ability of HSCs.

In a preferred embodiment of the present application, wherein the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

Tubulin inhibitors can be divided into tubulin polymerization inhibitors and tubulin formation inhibitors according to the mechanism. The tubulin polymerization inhibitors include Fosbretabulin (Combretastatin A4Phosphate (CA4P)) Disodium, SSE15206, Nocodazole, Lexibulin (CYT997), Colchicine, Maytansinol, EGFR inhibitors, Cucurbitacin B, Cabazitaxel, ABT -751(E7010), Vinblastine sulfate, Vincristine sulfate, Vincristine sulfate, Vindesinesulfate, Parbendazole, 2-Methoxyestradiol (2-MeOE2), Vindoline, 4-Demethylepipodophyllotoxin (NSC-122819, VM-26), Albendazole Oxide, Ansamitocin p-3 (Maytansinolisobutyrate, NSC292222), Plinabulin (NPI-2358), MMAF (Monomethylauristatin F), Vinorelbine Tartrate, Indibulin, etc.

Tubulin formation inhibitors include Monomethyl auristatin E (MMAE), CW069, Combretastatin A4, SKLB-23bb, Mebendazole, Carbendazim, Griseofulvin, H-Cys(Trt)-OH, ELR-510444, Trigonelline (N-Methylnicotinate), MMAD, etc.

Lexibulin, also known as CYT997, is a potent inhibitor of microtubule polymerization that induces rapid reorganization of microtubules. It inhibits the formation of the spindle and causes the cell cycle to arrest in the G2/M phase, inhibits tubulin, hinders the formation of blood vessels, and has potential anti-tumor activity.

Vinblastine sulfate (also known as NSC49842) is a vinca alkaloid. It can bind to tubulin on the spindle, causing microtubule crystallization, thereby inhibiting microtubule formation.

Colchicine is a microtubule polymerization inhibitor and an alkaloid drug. It was first approved by the FDA in 1961. It is extracted from the lily plant Colchicum autumnale. Colchicine binds to tubulin to form a complex,can prevent microtubule growth at low concentrations and can cause depolymerization of microtubules at high concentrations.

Nocodazole, a rapidly reversible inhibitor of microtubule polymerization, also inhibits Abl, E255K and T3151. It specifically binds to β-tubulin and affects the assembly and depolymerization of tubulin.

In a preferred embodiment of the present application, wherein the compound is used at a concentration of 1 nM-100 µM, preferably 0.1-10 µM, for example, the compound may be used at a concentration of 0.1 µM, 0.2 µM, 0.3 µM, 0.4µM, 0.5µM, 0.6µM, 0.7µM, 0.8µM, 0.9µM, 1µM, 2µM, 3µM, 4µM, 5µM, 6µM, 7µM, 8µM, 9µM, 10µM, 20µM, 30µM, 40µM, 50µM, 60µM, 70µM, 80 µM, 90 µM, 100 µM, or any range therebetween.

The present application provides use of a compound in the improvement ofthe transplantation efficiency of hematopoietic stem cells, wherein the compound is one or more selected from:a tubulin polymerization inhibitor, LND-212854, AZD0364, SCH772984, Pimasertib, Trametinib, IWR-1-endo, TTNPB, JNK -inhibitor IX and CHIR99021; preferably the compound is a tubulin polymerization inhibitor.

In a preferred embodiment of the present application, wherein the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

The transplantation efficiency of hematopoietic stem cells refers to the ability of hematopoietic stem cells from human donor to effectively migrate to the recipient's bone marrow after intravenous injection into the recipient, maintain growth in the bone marrow microenvironment, and play a role in repopulating of the hematopoietic system. To verify the transplantation efficiency of hematopoietic stem cells in the experiment, human hematopoietic stem cells are transplanted into an immune-deficient mouse model, the blood and immune organs of the recipient mice are detected by flow cytometry, and the content of human donor cells (hCD45 positive cell ratio) is anylyzed.

In a preferred embodiment of the present application, the compound is used at a concentration of 1 nM-100 µM, preferably 0.1-10 µM.

The present application provides a method for increasing CD184 protein expression on the surface of hematopoietic stem cells in a subject, comprising administering a compound to a subject in need thereof, wherein the compound is one or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;

Preferably, the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

In a preferred embodiment of the present application, the compound is used at a concentration of 1 nM-100 µM, preferably 0.1-10 µM.

The present application provides a method for improving transplantation efficiency of hematopoietic stem cells in a subject, comprising administering to a subject in need thereof a compound, wherein the compound is one or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;

Preferably, the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

In a preferred embodiment of the present application, the compound is used at a concentration of 1 nM-100 µM, preferably 0.1-10 µM.

By using the above-mentioned compounds, the present application can significantly increase the expression level of surfaceprotein CD184, thereby enhancing the migration ability of HSPCs and promoting the transplantation efficiency.

The present application provides use of the above-mentioned compounds in the preparation of a medicamentfor treatinga hematological malignant tumor, a hematological non-malignant tumor, a solid tumor, an immune system disease, a genetic or metabolic disease.

Preferably, for example, the hematological malignant tumor can be chronic myeloid leukemia, acute myeloid leukemia, acute lymphocytic leukemia, chronic myeloid leukemia, chronic lymphocytic leukemia, acute promyelocyticleukemia, non-Hodgkin's lymphoma, Hodgkin's lymphoma, myeloma, multiple myeloma, myelofibrosis and myelodysplastic syndrome, Burkitt's lymphoma, B-Cell lymphoma, follicular lymphoma, mantle cell lymphoma, T-cell lymphoma, plasmablastic lymphoma, cutaneous T-cell lymphoma, primary macroglobulinemia, plasma cell leukemia, plasmablastic lymphoma, hairy cell leukemia, systemic mastocytosis,blasticplasmacytoid dendritic cell neoplasm or other hematological malignant tumors.

The hematological non-malignant tumor can be, for example, aplastic anemia, Fanconianemia, thalassemia, sickle cell anemia, myelofibrosis, severe paroxysmal nocturnal hemoglobinuria, amegakaryocytic thrombocytopenia or other non-malignant blood systemic tumors.

The solid tumor can be breast cancer, ovarian cancer, testicular cancer, kidney cancer, neuroblastoma, small cell lung cancer, germ cell tumor, Ewing's sarcoma, soft tissue sarcoma, Wilms tumor, osteosarcoma, medulloblastoma, malignant brain tumor or other solid tumors.

The immune system disease can be severe combined immunodeficiency, severe autoimmune disease, primary central nervous system lymphoma, eczema thrombocytopenia with immunodeficiency syndrome, chronic granulomas, IPEX (Immune dysregulation, polyendocrinopathy, enteropathy, X-linked syndrome) syndrome, AL amylosis, POEMS syndrome, hemophagocytic syndrome, rheumatoid arthritis, multiple sclerosis, systemic sclerosis, systemic lupus erythematosus, Crohn's disease, polymyositis, dermatomyositis or other autoimmune, immune disorder or immunodeficiency diseases.

The genetic or metabolic disease can be mucopolysaccharidosis, dyskeratosiscongenita, lysosomal metabolic disease, spheroid leukoencephalopathy, metachromatic leukodystrophy, X-linked adrenoleukodystrophy, or other genetic diseases or metabolic diseases.

The present applicationprovides a medicamentfor treatinga hematological malignant tumor, a hematological non-malignant tumor, a solid tumor, an immune system disease, or a genetic or metabolic disease, comprising a compound which is one or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
preferably, the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

In a preferred embodiment of the present application, wherein the hematological malignant tumor can be chronic myeloid leukemia, acute myeloid leukemia, acute lymphocytic leukemia, chronic myeloid leukemia, chronic lymphocytic leukemia, acute promyelocyticleukemia, non-Hodgkin's lymphoma, Hodgkin's Lymphoma, myeloma, multiple myeloma, myelofibrosis and myelodysplastic syndromes, Burkitt lymphoma, B-cell lymphoma, follicular lymphoma, mantle cell lymphoma, T-cell lymphoma, plasmablastic lymphoma, cutaneous T-cell lymphoma, primary macroglobulinemia, plasma cell leukemia, plasmablastic lymphoma, hairy cell leukemia, systemic mastocytosis, blasticplasmacytoid dendritic cell neoplasm or other hematological malignant tumors.

The hematological non-malignant tumor can be, for example, aplastic anemia, Fanconianemia, thalassemia, sickle cell anemia, myelofibrosis, severe paroxysmal nocturnal hemoglobinuria, amegakaryocytic thrombocytopenia or other non-malignant blood systemic tumors.

The solid tumor can be breast cancer, ovarian cancer, testicular cancer, kidney cancer, neuroblastoma, small cell lung cancer, germ cell tumor, Ewing's sarcoma, soft tissue sarcoma, Wilms tumor, osteosarcoma, medulloblastoma, malignant brain tumor or other solid tumors.

The immune system diseases can be severe combined immunodeficiency, severe autoimmune disease, primary central nervous system lymphoma, eczema thrombocytopenia with immunodeficiency syndrome, chronic granulomas, IPEX (immune dysregulation, polyendocrinopathy, enteropathy, X-linked syndrome) syndrome, AL amylosis, POEMS syndrome, hemophagocytic syndrome, rheumatoid arthritis, multiple sclerosis, systemic sclerosis, systemic lupus erythematosus, Crohn's disease, polymyositis, dermatomyositis or other autoimmune, immune disorder or immunodeficiency diseases.

The genetic or metabolic disease can be mucopolysaccharidosis, dyskeratosiscongenita, lysosomal metabolic disease, spheroid leukoencephalopathy, metachromatic leukodystrophy, X-linked adrenoleukodystrophy, or other genetic disease or metabolic diseases.

The present application provides use of a compound in the preparation of a medicament for treating a disease associated with the low expression level of surfaceprotein CD184,wherein the compound is one or more selected from the group consisting of: a tubulin polymerization inhibitor, LND-212854, AZD0364, SCH772984, Pimasertib, Trametinib , one or more of IWR-1-endo, TTNPB, JNK-inhibitor IX and CHIR99021; preferably the compound is a tubulin polymerization inhibitor;
preferably, the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

The present application provides use of a compound for the preparation of a medicament for treating a disease associated with low transplantation efficiency level of hematopoietic stem cells, wherein the compound is one or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferablythe compound is a tubulin polymerization inhibitor;
preferably, the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

The present application provides use of a compound in the improvement ofthe homing ability of hematopoietic stem cells, wherein the compound is one, or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferablythe compound is a tubulin polymerization inhibitor;
preferably, the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

The homing ability of hematopoietic stem cells refers to the ability of human donor hematopoietic stem cells to effectively colonize the recipient's bone marrow after intravenous injection into the recipient and play a role in repopulating of the hematopoietic system. To verify the homing ability of hematopoietic stem cells in the experiment, human hematopoietic stem cells aretransplanted into an immunodeficient mouse model, the bone marrow cells of the recipient mice are detected by flow cytometry, and the content of human donor cells (the ratio of hCD45 positive cells) is analyzed.

In a preferred embodiment of the present application, the compound is used at a concentration of 1 nM-100 µM, preferably 0.1-10 µM.

The present application provides a method for improving the homing ability of hematopoietic stem cells in a subject, comprising administering to a subject in need thereof a compound, wherein the compoundis one or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferablythe compound is a tubulin polymerization inhibitor;
preferably, the tubulin polymerization inhibitor is one or more selected from the group consisting of: Lexibulin, Vinblastine sulfate, Colchicine and Nocodazole; preferably the tubulin polymerization inhibitor is Lexibulin, Vinblastine sulfate and/or Colchicine.

In a preferred embodiment of the present application, the compound is used at a concentration of 1 nM-100 µM, preferably 0.1-10 µM.

Wherein the subject refers to any organism that may be affected by the above-mentioned conditions, preferably a mammal, such as a human, a mouse, and the like.

The hematopoietic stem cells can be hematopoietic stem cells from any source, such as hematopoietic stem cells derived from cord blood, hematopoietic stem cells derived from recruited peripheral blood, hematopoietic stem cells derived from bone marrow, and the like.

The hematopoietic stem cells can be, for example, frozen hematopoietic stem cells or fresh hematopoietic stem cells.

The compound provided by the present application can increase the expression of protein CD184 on thesurface of hematopoietic stem cells through short-time treatment *in vitro,* without affecting the phenotype, viability and various characteristics of the cells. Through the binding of the chemokine receptor CD184 expressed by HSC to the chemokine ligand SDF1 in the bone marrow microenvironment, the migration ability and homing ability of HSCs in the bone marrow are improved, and the transplantation efficiency and therapeutic effect of HSCs are enhanced. By using the compound described above, the hematopoietic stem cells have safety, good viability and sufficient cell quantity, so as to maintain the effectiveness of long term repopulating of the hematopoietic system after hematopoietic stem cell transplantation.

### EXAMPLES

The present application generally and/or specifically describes the materials and assays used in the test. In the following examples, unless otherwise specified, % represents wt%, that is, weight percentage. The reagents or instruments used without indicating the manufacturer are all conventional reagent products that can be purchased from the market, wherein, Table 1 is the table of the raw materialsource used in the examples.

**Table 1. The table of raw material source used in the examples**

| Materialname | Purity | Catalog | Batch number | Manufacturers |
|---|---|---|---|---|
| LND-212854 | 100.00% | S7147 | S714701 | SELLECK |
| AZD0364 | 99.95% | S8708 | S870801 | SELLECK |
| SCH772984 | 99.07% | S7101 | S710104 | SELLECK |
| Pimasertib | 99.37% | S1475 | S147504 | SELLECK |
| Trametinib | 99.51% | S2673 | S267309 | SELLECK |
| IWR-1-endo | 99.01% | S7086 | S708602 | SELLECK |
| TTNPB | 99.65% | S4627 | S462701 | SELLECK |
| JNK-inhibitor IX | 99.91% | S7508 | S750801 | SELLECK |
| CHIR99021 | 99.01% | S2924 | S292406 | SELLECK |
| Lexibulin | 99.37% | S2195 | S219502 | SELLECK |
| Vinblastine sulfate | 97.70% | S4505 | S450502 | SELLECK |
| Colchicine | 99.44% | S2284 | S228403 | SELLECK |
| Nocodazole | 99.07% | S2775 | S277501 | SELLECK |
| M344 | 99.36% | S2779 | S277901 | SELLECK |

### Example 1Isolation of hematopoietic stem cells

The human hematopoietic stem cells used were mainly derived from umbilical cord blood. After the umbilical cord blood samples were retrieved from the hospital, they were placed at room temperature for 0.5-1 hour, and then the blood samples were transferred from the blood collection bag to a 50ml centrifuge tube and mixed gently. 100µl samples were taken with a pipette for counting white blood cells and measuring the positive proportion of CD34 cellsby flow cytometry. The remaining blood samples were diluted 1:1 with normal saline containing 1% HAS and mixed gently by a pipette. The mixture was placed in a new 50ml centrifuge tube, an appropriate amount of lymphocyte separation medium was added to the bottom of the tube, and they were centrifuged at 400g, accelerated at 3, and decelerated at 0, for 30 minutes. After centrifugation, the centrifuge tube was slowly taken out to keep the liquid level even. The buffy coat cells were carefully sucked with a pipette and transferred to a new centrifuge tube. The cells were washed with 1% HAS saline, and centrifuged at 500 g, accelerated at 9, and decelerated at 5, for 10 minutes.

After centrifugation, the cells were resuspended and CD34 positive cells were sorted (CD34+ Cell Magnetic Bead Sorting Kit, Miltenyi, 130-046-703).

First, for per 1×10⁶ cells in total, 100 µlFcR Blocking Antibody and 100 µl CD34-Beads were added, and incubated at 4°C for 30 minutes. After incubation, they were washed with 40ml physiological saline containing 1% HAS, centrifuged at 400g for 8 minutes, and resuspended at 1×10⁹ cells per 3ml.

The CD34+ cell sorting column was placed on the MACS magnetic separation rack, a 40 µm cell sieve was placed on the sorting column, and a 15 ml centrifuge tube was placed under the sorting column. The filter sieve was rinsed with 3 ml of physiological saline containing 1% HAS, and then the cell suspension was added. After the dropping was completed, the filter was washed 3 times with 3 ml of physiological saline containing 1% HAS. The magnetic field was removed from the adsorption column, 1% HAS saline was added to collect the cells into a centrifuge tube. It was repeatedonce,cells were mixed, counted, and cultured or frozen. A portion of cells were collected for detecting CD34 expression by flow cytometry to confirm its purity.

### Example 2 Culture of hematopoietic stem cells

First, a complete medium for hematopoietic stem cells was prepared, and its components and usage are as follows:

**Table 2. Table of ingredients used in the complete medium for hematopoietic stem cells**

| | Dosage | Brand | Catalog |
|---|---|---|---|
| StemSpanSFMEII | | Stem cell | 09655 |
| IL6 | 20ng/ml | PeproTech | 200-06 |
| TPO | 100ng/ml | PeproTech | 300-18-100 |
| FLT3L | 100ng/ml | PeproTech | AF-300-19-100 |
| SCF | 100ng/ml | PeproTech | 300-07-100 |

According to the counting results in Example 1, 5×10⁴CD34+ cells were added to each well of a 24-well plate, and the cells were cultured with 1 ml of complete medium per well for 24 hours.

The next day, the compound was dissolved in DMSO and mixed, and then diluted with PBS to a specific concentration (see Table 3). An equal volume of 10 µl of the diluted compound per well was added to the cell culture medium, mixed well, and placed in an incubator for 16 hours.

**Table 3: The working concentration of the compound used in the Examples.**

| | High Concentration | Medium Concentration | Low Concentration |
|---|---|---|---|
| Lexibulin | 10µM | 1µM | 100nM |
| Vinblastine sulfate | 10µM | 1µM | 100nM |
| M344 | 5µM | 1µM | 0.5µM |
| Colchicine | 1µM | 100nM | 10nM |
| LDN-212854 | 50µM | 5µM | 0.5µM |
| AZD0364 | 1µM | 100nM | 10nM |
| SCH772984 | 100nM | 10nM | 1nM |
| Trametinib | 250nM | 50nM | 10nM |
| IWR-1-endo | 25µM | 5µM | 1µM |
| TTNPB | 5µM | 1µM | 0.2µM |
| Pimasertib | 50µM | 5µM | 0.5µM |
| Nocodazole | 100µM | 10µM | 1µM |
| JNK-inhibitor IX | 50µM | 5µM | 0.5µM |
| CHIR-99021 | 10µM | 3µM | 1µM |

### Example 3 Detection of CD184 protein expression

On the third day, an antibody pre-mixed solution was prepared: a solution of PBS+0.5% BSA was pre-mixed with fluorescent antibody BV510 conjugated anti-human CD34 (Biolegend clone 581), FITC conjugated anti-human CD90 (Biolegend, clone 5E10) and APCCy7 conjugated anti-human CD45RA (Biolegend, clone HI100), 50 µl/ml of each antibody was added, and stored in the dark.

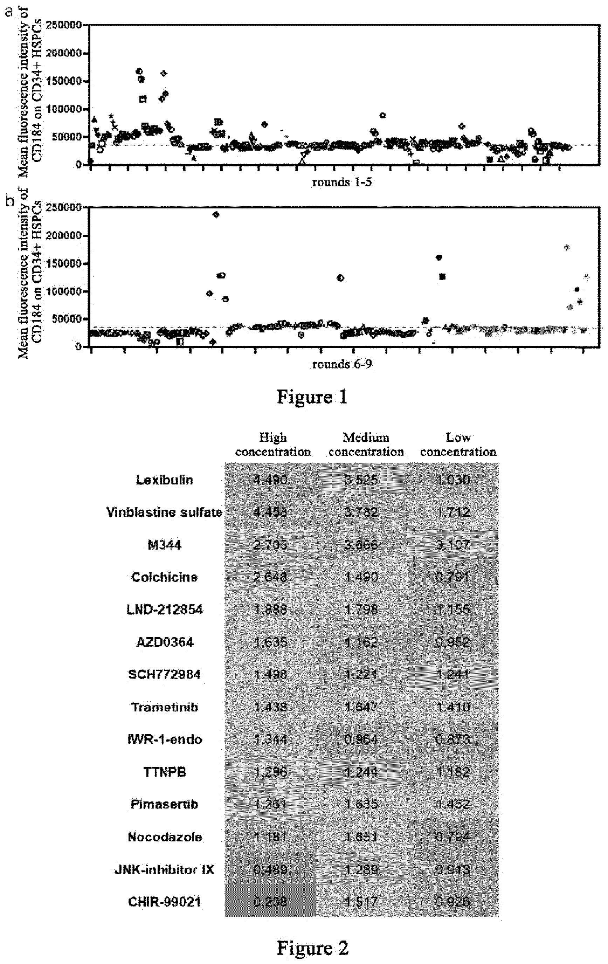

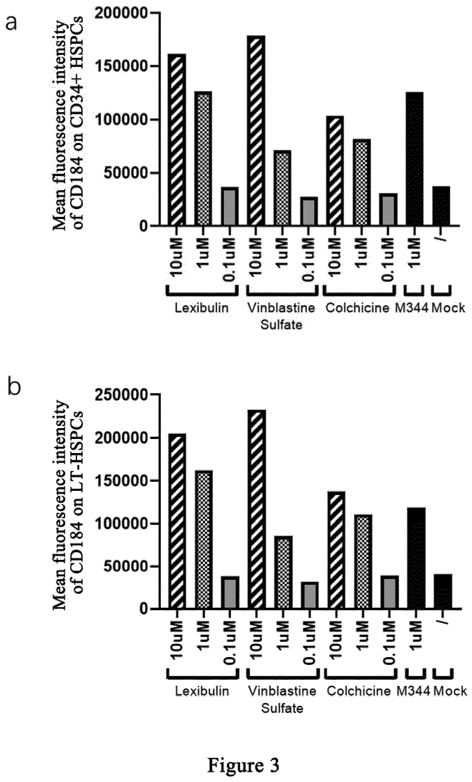

Cells were collected into centrifuge tubes, centrifuged at 400g for 5 minutes.The supernatant was removed, added with 50 µl of antibody pre-mixed solution (containing 2.5 µl of each antibody) in each sample, resuspended and incubated at 4°C for 20 minutes in the dark. The cells were washed with 1 ml of PBS added to each tube, centrifuged at 400g for 5 minutes and resuspended with 50 µl of PBS+0.5% BSA solution. 2.5 µl of PE-conjugated anti-CD184 antibody (Biolegend, clone 12G5) was added to each sample, incubated at 37°C for 30 minutes, washed, and stained with 7AAD to identify cell viability. Flow cytometry (Beckman Coulter CytoFLEX) was used to detect cell surface fluorescence, and the mean fluorescence intensity (MFI) and positive ratio of CD184 expression in HSPCs and LT-HSCs were analyzed, respectively. Among them, HSPCs are CD34+ cells, and LT-HSCs are CD34+CD90+CD45RA-cells.
(1)Preliminary screening of compounds: 139 compounds were used for preliminary screening, among which 89 compounds were screened in rounds 1-5, and 50 compounds were screened in rounds 6-9. During the *in vitro* culture of CD34+ HSPCs, they were treated for 16 hours by adding three concentrations of compounds to the complete medium. Then the mean fluorescence intensity of surface protein CD184 expression was detected by flow cytometry.The results are shown in Figure. 1.
   The results show that the tubulin polymerization inhibitor, LND-212854, AZD0364, SCH772984, Pimasertib, Trametinib, IWR-1-endo, TTNPB, JNK-inhibitor IX and CHIR99021 can enhance the expression of CD184 in CD34+HSPCs. The tubulin polymerization inhibitors are Lexibulin, Vinblastine sulfate, Colchicine and Nocodazole.
(2) Secondary screening of compounds: the 13 compounds obtained from the preliminary screening were further verified using three concentrations of high, medium and low, with three replicates for each concentration. After 16 hours of treatment with the above-mentioned compounds, the average fluorescence intensity of surface protein CD184 expression was detected by flow cytometry.The result is shown in Figure. 2. Wherein, the data in the figure are the ratio of the mean CD184 fluorescence intensity of each group to the control group (Mock). (M344 is the positive control group; Mock is the negative control group untreated during culture.)
   It can be seen from Figure 2 that when the compound is at a high concentration, Lexibulin and Vinblastine sulfate can significantly increase the expression of CD184, which is nearly 2 times that of the positive control M344 and more than 4 times that of the negative control; and Colchicine has comparable effects to M344M344, and is 2.6 times that of the negative control at high concentration, indicating that Lexibulin, Vinblastine sulfate and Colchicine can significantly improve the expression ability of CD184.
(3)Effects of tubulin polymerization inhibitors Lexibulin, Vinblastine sulfate and Colchicine on the expression of CD184
   After treating the above-mentioned tubulin polymerization inhibitors at three concentrations (10 µM, 1 µM, 0.1 µM) for 16 hours, mean fluorescence intensity (MFI) of CD184 on CD34+HSPCs (a), and CD34+CD90+CD45RA-LT- LT-HSCs(b) were detected by flow cytometry.The results are shown in Figure 3.

### Example 4 Detection of cell viability

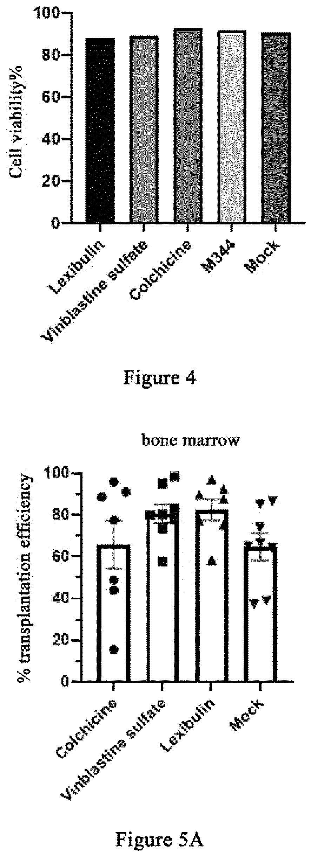

On the third day, the cells treated with Lexibulin, Vinblastine sulfate, Colchicine, M344 and Mock in the culture platesof Example 2 were collected, and centrifuged at 400g for 5 minutes. The supernatant was removed. The cells were resuspended with PBS.20 µl of diluted cell samples were taken, and 20 µl AOPI staining solution (Nexcelom Bioscience, CS2-0106) in equal proportion was added and mixed thoroughly. 20 µl of the mixed solution was added to the sample well of the counting plate, and the cell counting and cell viability were detected on a fluorescent cell counter (Nexcelom, Cellometer K2). The assay results are shown in Figure 4.

### Example 5

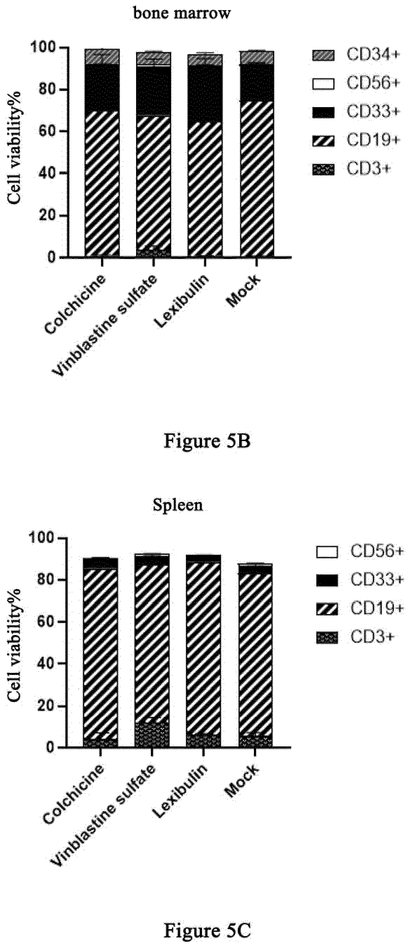

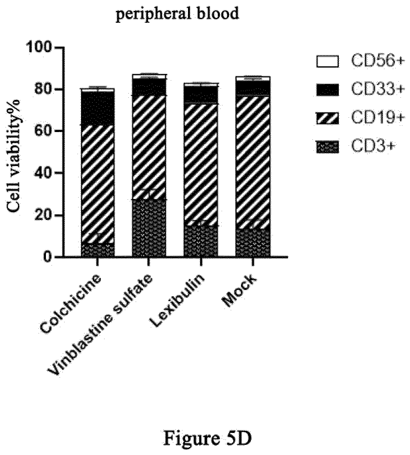

Human CD34+ HSPCs treated with Colchicine, Vinblastine sulfate or Lexibulin for a short time, or those in the control group were transplanted into NPG (NOD-scid Il2rg-/-) immunodeficient mice irradiated with 1.0 Gy, 8 mice in each group. 17 weeks after transplantation, cell transplantation efficiency in mouse bone marrow, and distribution proportion of human T cells labeled with human CD3, CD4 and CD8 antibodies, myeloid cells labeled with human CD33 antibody, B cells labeled with CD19 antibody, NK cells labeled with human CD56 antibody, and hematopoietic stem and progenitor cells labeled with human CD34 antibody in bone marrow, peripheral blood, and spleen were detected. The results are shown in Figure 5A to Figure 5D, where the transplantation efficiency = hCD45⁺proportion/(hCD45⁺proportion+mCD45⁺proportion)x100%

The results in Figure 5A show that for the HSPCs treated with small molecule Colchicine, Vinblastine sulfate or Lexibulin17 weeks after transplantation, compared with the control group, the transplantation efficiency of cells in the bone marrow of NPG mice is increased, wherein the Colchicine group is 65.84% the Vinblastine sulfate groupis 80.73%, theLexibulin group is82.46%, and theMock group is 64.7%.

The results in Figure 5B to Figure 5D show that HSPCs treated with small molecule Colchicine, Vinblastine sulfate or Lexibulin can differentiate into T cells, myeloid cells, B cells and NK cells in bone marrow, spleen and peripheral blood, indicating that HSPCs treated with small molecules still retain the ability of hematopoietic stem cells to repopulate the hematopoietic system.

The above are only preferred embodiments of the present application, and are not intended to limit the present application in other forms. Any person skilled in the art may use the technical content disclosed above to make changes or modifications to obtain equivalent embodiments with equivalent changes. However, without departing from the content of the technical solutions of the present application, any simple modifications, equivalent changes and modifications made to the above embodiments according to the technical essence of the present application still belong to the protection scope of the technical schemes of the present application.

## Claims

1. Use of a compound in the improvement of CD184 protein expression on the surface of hematopoietic stem cells, wherein the compoundis one or more selected from the group consisting of: a tubulin polymerization inhibitor, LND-212854, AZD0364, SCH772984, pimasertib, trametinib, IWR-1-endo, TTNPB, JNK-inhibitor IX and CHIR99021.

2. The use according to claim 1, wherein the compound is a tubulin polymerization inhibitor, preferably, the tubulin polymerization inhibitor is one or more selected from the group consisting of:lexibulin, vinblastine sulfate, colchicine and nocodazole.

3. The use according to claim 2, wherein the tubulin polymerization inhibitor is lexibulin, vinblastine sulfate and/or colchicine.

4. The use according to any one of claims 1-3, wherein the compound is used at a concentration of 1 nM-100 µM.

5. Use of a compound in the improvement of transplantation efficiency of hematopoietic stem cells, wherein the compoundis one or more selected from the group consisting of: a tubulin polymerization inhibitor, LND-212854, AZD0364, SCH772984, pimasertib, trametinib, IWR-1-endo, TTNPB, JNK-inhibitor IX and CHIR99021; preferablythe compound is a tubulin polymerization inhibitor.

6. The use according to claim 5, wherein the tubulin polymerization inhibitor is one or more selected from the group consisting of:lexibulin, vinblastine sulfate, colchicine and nocodazole, preferably, the tubulin polymerization inhibitor is lexibulin, vinblastine sulfate and/or colchicine.

7. The use according to claim 5 or 6, wherein the compound is used at a concentration of 1 nM-100 µM.

8. A method for improving CD184 protein expression on the surface of hematopoietic stem cells ina subject, comprising supplementing a compound to a medium for culturing the hematopoietic stem cells, wherein the compoundis one or more selected from the group consisting of:a tubulin polymerization inhibitor, LND-212854, AZD0364, SCH772984, pimasertib, trametinib, IWR-1-endo, TTNPB, JNK-inhibitor IX and CHIR99021; preferablythe compound is a tubulin polymerization inhibitor;
preferably, the tubulin polymerization inhibitoris one or more selected from the group consisting of:lexibulin, vinblastine sulfate, colchicine and nocodazole; preferably the tubulin polymerization inhibitor is lexibulin, vinblastine sulfate and/or colchicine.

9. The method according to claim 8, wherein the compound is used at a concentration of 1 nM-100 µM.

10. A method for improving transplantation efficiency of hematopoietic stem cells in a subject, comprising supplementing a compound to a medium for culturing the hematopoietic stem cells, wherein the compound is oneor more selected from the group consisting of: a tubulin polymerization inhibitor, LND-212854, AZD0364, SCH772984, pimasertib, trametinib, IWR-1-endo, TTNPB, JNK-inhibitor IX and CHIR99021; preferablythe compound is a tubulin polymerization inhibitor;
preferably, the tubulin polymerization inhibitoris one or more selected from the group consisting of:lexibulin, vinblastine sulfate, colchicine and nocodazole; preferably the tubulin polymerization inhibitor is lexibulin, vinblastine sulfate and/or colchicine.

11. The method according to claim 10, wherein the compound is used at a concentration of 1 nM-100 µM.

12. Use of a compound in the preparation of a medicamentfor treatinga hematological malignant tumor, a hematological non-malignant tumor, a solid tumor, an immune system disease, a genetic or metabolic disease, wherein the compound is one or more selected from the group consisting of: a tubulin polymerization inhibitor, LND-212854, AZD0364, SCH772984, pimasertib, trametinib, IWR-1-endo, TTNPB, JNK-inhibitor IX and CHIR99021; preferablythe compound is a tubulin polymerization inhibitor;
preferably, the tubulin polymerization inhibitoris one or more selected from the group consisting of:lexibulin, vinblastine sulfate, colchicine and nocodazole; preferably the tubulin polymerization inhibitor islexibulin, vinblastine sulfate and/or colchicine.

13. The use according to claim 12, wherein the hematological malignant tumor is chronic myeloid leukemia, acute myeloid leukemia, acute lymphocytic leukemia, chronic myeloid leukemia, chronic lymphocytic leukemia, acute promyelocyticleukemia, non-Hodgkin's lymphoma, Hodgkin's lymphoma, myeloma, multiple myeloma, myelofibrosis and myelodysplastic syndrome, Burkitt'slymphoma, B-cell lymphoma, follicular lymphoma, mantle cell lymphoma, T-cell lymphoma, plasmablastic lymphoma, cutaneous T-cell lymphoma, primary macroglobulinemia, plasma cell leukemia, plasmablastic lymphoma, hairy cell leukemia, systemic mastocytosisor blasticplasmacytoid dendritic cellneoplasm;
thehematological non-malignant tumor is aplastic anemia, Fanconianemia, thalassemia, sickle cell anemia, myelofibrosis, severe paroxysmal nocturnal hemoglobinuria or amegakaryocyticthrombocytopenia;
the solid tumor is breast cancer, ovarian cancer, testicular cancer, kidney cancer, neuroblastoma, small cell lung cancer, germ cell tumor, Ewing's sarcoma, soft tissue sarcoma, Wilms tumor, osteosarcoma, medulloblastoma or malignant brain tumor;
the immune system disease is severe combined immunodeficiency, severe autoimmune disease, primary central nervous system lymphoma, eczema thrombocytopenia with immunodeficiency syndrome, chronic granuloma, IPEX syndrome, AL amylosis, POEMS syndrome, hemophagocytic syndrome, rheumatoid arthritis, multiple sclerosis, systemic sclerosis, systemic lupus erythematosus, Crohn's disease, polymyositis, or dermatomyositis; and
the genetic or metabolic disease ismucopolysaccharidosis, dyskeratosiscongenita, lysosomal metabolic disease, spheroid leukoencephalopathy, metachromatic leukodystrophy, or X-linked adrenoleukodystrophy.

14. A medicament for treatinga hematological malignant tumor, a hematological non-malignant tumor, a solid tumor, an immune system disease, a genetic or metabolic disease, comprising a compound, wherein the compoundisone or more selected from the group consisting of: a tubulin polymerization inhibitor, LND-212854, AZD0364, SCH772984, pimasertib, trametinib, IWR-1-endo, TTNPB, JNK-inhibitor IX and CHIR99021; preferably the compound is a tubulin polymerization inhibitor;
preferably, the tubulin polymerization inhibitor isone or more selected from the group consisting of:lexibulin, vinblastine sulfate, colchicine and nocodazole; preferably the tubulin polymerization inhibitor is lexibulin, vinblastine sulfate and/or colchicine.

15. The medicament according to claim 14, wherein the hematological malignant tumor is chronic myeloid leukemia, acute myeloid leukemia, acute lymphocytic leukemia, chronic myeloid leukemia, chronic lymphocytic leukemia, acute promyelocyticleukemia, non-Hodgkin's lymphoma, Hodgkin's lymphoma, myeloma, multiple myeloma, myelofibrosis and myelodysplastic syndrome, Burkitt'slymphoma, B-Cell lymphoma, follicular lymphoma, mantle cell lymphoma T-cell lymphoma, plasmablastic lymphoma, cutaneous T-cell lymphoma, primary macroglobulinemia, plasma cell leukemia, plasmablastic lymphoma, hairy cell leukemia, systemic mastocytosis or blasticplasmacytoid dendritic cellneoplasm;
thehematological non-malignant tumor is aplastic anemia, Fanconianemia, thalassemia, sickle cell anemia, myelofibrosis, severe paroxysmal nocturnal hemoglobinuria or amegakaryocytic thrombocytopenia;
the solid tumor is breast cancer, ovarian cancer, testicular cancer, kidney cancer, neuroblastoma, small cell lung cancer, germ cell tumor, Ewing's sarcoma, soft tissue sarcoma, Wilms tumor, osteosarcoma, medulloblastoma or malignant brain tumor;
the immune system disease is severe combined immunodeficiency, severe autoimmune disease, primary central nervous system lymphoma, eczema thrombocytopenia with immunodeficiency syndrome, chronic granuloma, IPEX syndrome, AL amylosis, POEMS syndrome, hemophagocytic syndrome, rheumatoid arthritis, multiple sclerosis, systemic sclerosis, systemic lupus erythematosus, Crohn's disease, polymyositis, or dermatomyositis; andthe genetic or metabolic disease is mucopolysaccharidosis, dyskeratosiscongenita, lysosomal metabolic disease, spheroid leukoencephalopathy, metachromatic leukodystrophy, or X-linked adrenoleukodystrophy.

16. Use of a compound in the preparation of a medicamentfor treating a diseaseassociated with low expression level of surface protein CD184, wherein the compoundisone or more selected from the group consisting of: a tubulin polymerization inhibitor, LND-212854, AZD0364, SCH772984, pimasertib, trametinib, IWR-1-endo, TTNPB, JNK-inhibitor IX and CHIR99021; preferablythe compound is a tubulin polymerization inhibitor;
preferably, the tubulin polymerization inhibitorisone or more selected from the group consisting of:lexibulin, vinblastine sulfate, colchicine and nocodazole; preferably the tubulin polymerization inhibitor is lexibulin, vinblastine sulfate and/or colchicine.

17. Use of a compound in the preparation of a medicamentfor treating a disease associated with low transplantation efficiency level of hematopoietic stem cells, wherein the compoundis one or more selected from the group consisting of: a tubulin polymerization inhibitor, LND-212854, AZD0364, SCH772984, pimasertib, trametinib, IWR-1-endo, TTNPB, JNK-inhibitor IX and CHIR99021; preferablythe compound is a tubulin polymerization inhibitor;
preferably, the tubulin polymerization inhibitor is one or more selected from the group consisting of:lexibulin, vinblastine sulfate, colchicine and nocodazole; preferably the tubulin polymerization inhibitor is lexibulin, vinblastine sulfate and/or colchicine.

18. Use of a compound in the improvementof the homing ability of hematopoietic stem cells, wherein the compound is one or more selected from the group consisting of: atubulin polymerization inhibitor, LND-212854, AZD0364, SCH772984, pimasertib, trametinib, IWR-1-endo, TTNPB, JNK-inhibitor IX and CHIR99021; preferablythe compound is a tubulin polymerization inhibitor;
preferably, the tubulin polymerization inhibitoris one or more selected from the group consisting of:lexibulin, vinblastine sulfate, colchicine and nocodazole; preferably the tubulin polymerization inhibitor is lexibulin, vinblastine sulfate and/or colchicine.

19. The use according to claim 18, wherein the compound is used at a concentration of 1 nM-100 µM.

20. A method for improving the homing ability of hematopoietic stem cells in a subject, comprising supplementing a compound to a medium for culturing the hematopoietic stem cells, wherein the compound is one or more selected from the group consisting of: a tubulin polymerization inhibitor, LND-212854, AZD0364, SCH772984, pimasertib, trametinib, IWR-1-endo, TTNPB, JNK-inhibitor IX and CHIR99021; preferablythe compound is a tubulin polymerization inhibitor;
preferably, the tubulin polymerization inhibitor is one or more selected from the group consisting of:lexibulin, vinblastine sulfate, colchicine and nocodazole; preferably the tubulin polymerization inhibitor is lexibulin, vinblastine sulfate and/or colchicine.

21. The method according to claim 20, wherein the compound is used at a concentration of 1 nM-100 µM.

22. Use of a compound in the preparation of a medicament for treating a disease associated with low homing ability level of hematopoietic stem cells, wherein the compound is one or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferablythe compound is a tubulin polymerization inhibitor;
preferably, the tubulin polymerization inhibitor is one or more selected from the group consisting of: lexibulin, vinblastine sulfate, colchicine and nocodazole; preferably the tubulin polymerization inhibitor islexibulin, vinblastine sulfate and/or colchicine.

23. A cell culture medium comprising a compound, wherein thecompoundis one, two, three, four, or more selected from the group consisting of: a tubulin polymerization inhibitor, LND-212854, AZD0364, SCH772984, Pimasertib, Trametinib, IWR-1-endo, TTNPB, JNK-inhibitor IX and CHIR99021; preferably the compound is a tubulin polymerization inhibitor;
preferably, the tubulin polymerization inhibitor is one, two, three, or four selected from the group consisting of: lexibulin, vinblastine sulfate, colchicine and nocodazole; preferably the tubulin polymerization inhibitor is lexibulin, vinblastine sulfate and/or colchicine.

24. The cell culture medium according to claim 23, wherein the cell culture medium is a hematopoietic stem cell culture medium.

25. The culture medium according to claim 23 or 24, wherein the compound is present in the culture medium at a concentration of 1 nM-100 µM, e.g., 10 nM-50 µM, 0.1 µM-20 µM, or 1 µM-10 µM, such as 0.1 µM, 0.2 µM, 0.3 µM , 0.4µM, 0.5µM, 0.6µM, 0.7µM, 0.8µM, 0.9µM, 1µM, 2µM, 3µM, 4µM, 5µM, 6µM, 7µM, 8µM, 9µM, 10µM, 20µM, 30µM, 40µM, 50µM, 60µM, 70µM , 80 µM, 90 µM, 100 µM, or any range therebetween.

26. A method for treating a hematological malignant tumor, a hematological non-malignant tumor, a solid tumor, an immune system disease, a genetic or metabolic disease in a subject, comprising administering to the subject hematopoietic stem cells, wherein the hematopoietic stem cells are hematopoietic stem cells cultured in a medium supplemented with a compound, wherein the compoundis one, two, three, four, or more selected from the group consisting of: a tubulin polymerization inhibitor, LND-212854, AZD0364, SCH772984, pimasertib, trametinib, IWR-1-endo, TTNPB, JNK-inhibitor IX and CHIR99021; preferably the compound is a tubulin polymerization inhibitor;
preferably, the tubulin polymerization inhibitor is one, two, three, or four selected from the group consisting of: lexibulin, vinblastine sulfate, colchicine and nocodazole; preferably the tubulin polymerization inhibitor is lexibulin, vinblastine sulfate and/or colchicine.

27. The method according to claim 26, wherein CD184 protein expression is increased after the hematopoietic stem cells are cultured in a medium supplemented with the compound.
